# EUROPEAN PATENT APPLICATION

(11) **EP 2 962 595 A1**
(43) Date of publication of application: **06.01.2016**
(21) Application number: 14175266.7
(22) Date of filing: 01.07.2014
(51) Int. Cl.: A45D 34/04

(54) **Device for treatment of a cutaneous surface or mucous membrane**

(71) Applicant: YouMedical Brands B.V., 1059 AT Amsterdam (NL)
(72) Inventor: Herweijer, Jan Philip, 1059 AT Amsterdam (NL); Witte, Johannes Hendricus, 1059 AT Amsterdam (NL); Kneppers, Job, 2613 PZ Delft (NL)
(74) Representative: De Vries & Metman

(57) **Abstract**

Method and device (1) for treatment of a cutaneous surface or a mucous membrane. The device comprises a container (2), such as an aerosol can, for containing a composition comprising at least one refrigerant. The container has a container outlet (3) and a valve communicating with the container outlet. An applicator (6) is mounted or mountable to the container. The applicator comprises a contact surface (8) and a dispensing channel (20) between the container outlet and one or more applicator outlets (22) arranged for wetting the contact surface. The contact surface is defined by a thermo-conductive porous heat exchanger (9). The dispensing channel defining a flow path crossing the heat exchanger. The contact surface can for example be formed by a non-porous layer, e.g. of plastic or rubber.

## Description

The invention relates to a device for treatment of a cutaneous surface or mucous membrane, e.g., for medical or cosmetic purposes. The device comprises a container containing a refrigerant and having an outlet for the refrigerant and a valve communicating with the outlet.

Devices containing a refrigerant can be used for cold treatment of disorders. For some treatments it may be desirable to combine such cold treatment with the application of an active ingredient. For example, US 2008/0142036 discloses a device for cooling a cutaneous surface after application of a cosmetic product. First the cosmetic product is applied onto the skin. Subsequently, the device is used for cooling the treated cutaneous surface.

The present invention has as one of its objects to provide a device for a simple and effective treatment of a cutaneous surface or mucous membrane.

The object is achieved with a device for treatment of a cutaneous surface, the device comprising:
a container for containing a composition comprising at least one refrigerant and at least one therapeutic or cosmetic ingredient, the container having a container outlet and a valve communicating with the container outlet,
an applicator mounted or mountable to the container, the applicator comprising a contact surface and a dispensing channel between the container outlet and an applicator outlet arranged for wetting the contact surface;
a thermo-conductive porous heat exchanger in the channel defining the contact surface, the dispensing channel defining a flow path crossing the heat exchanger.

This makes it possible to apply, e.g., a therapeutic or cosmetic ingredient or an excipient, by means of a cooled contact surface with a single applicator in a single massaging movement. It was also found that topical application of a therapeutic or cosmetic ingredient via a cooled contact surface can contribute to a better dispersion of the ingredients.

The container can for example be an aerosol can. It has been found that aerosol compositions have a longer shelf life than other carrier compositions, such as ointments, with the same therapeutic or cosmetic ingredients.

To prevent contamination of the porous material the heat exchanger may comprise a non-porous layer forming the contact surface. Such a non-porous layer can for example be made of a plastic or rubber material. Optionally, the non-porous layer can be textured or roughened at the contact surface. This makes the device suitable for scrubbing the skin to be treated during treatment.

The applicator may for example comprise an opening exposing the contact surface. The heat exchanger can be held by a ridge at least partly bordering the opening, the ridge being profiled to define the applicator outlets at the circumference of the contact surface. This enables effective fixating of the heat exchanger in the applicator housing without hindering access to the contact surface. The liquid is dispensed sideways over the contact surface.

If the contact surface is formed by a non-porous plastic or rubber layer, part or all of the outlets may be formed by slits in the non-porous layer which are opened by back pressure during dispensing.

In a specific embodiment the applicator comprises a bush having one end connected to the container outlet and one end engaging the porous heat exchanger. This way, the bush defines a channel section between the container outlet and the porous heat exchanger. The bush may comprise a support surface resting on top of the container outlet. Such a support surface can for example be formed by an inner ridge of the bush. This way, the valve, resiliently biased to its closing position, will resiliently push against the bush, which will in turn push against the heat exchanger. This prevents relative movement of the heat exchanger and the applicator housing.

To release the liquid the applicator may for example be moveable between a rest position with the valve being closed, and a dispensing position with the support surface of the bush pressing the container outlet down to open the valve. The valve may resiliently bias the applicator to the rest position.

In a more specific embodiment, the outer end of the bush engaging the heat exchanger comprises a collar with a circumferential edge sealing against an inner cylindrical skirt of the applicator. The circumferential edge can for example be clicked into a matching recess in an inner wall of the cylindrical skirt. The collar may for example be curved, e.g., as an inverse saucer shape, to resiliently press against the porous heat exchanger.

In a specific embodiment the applicator may comprise an outer skirt extending over a top edge of the container. The applicator may for example comprise a snap joint between the applicator and the container allowing up and down movement of the applicator between the rest position and the dispensing position.

The porous heat exchanger is made of a heat conductive material, e.g., of a sintered material, such as a metal alloy like brass, or a heat conductive plastic. The porous heat exchanger may for example be made of a material having a heat conductivity of at least 10 W⁻¹K⁻¹, e.g.. at least 20 W⁻¹K⁻¹. For low temperature cost effective heat exchangers (for example reaching temperatures of -10°C to -50°C), it is favourable to use a sintered metal for the porous member, for example sintered brass, although other metals having good heat conducting properties are conceivable, such as alloys of aluminium, copper, steel etc. Brass has the advantage that it also has a disinfective effect. The size and material properties of the sintered material make it possible to define the desired heat conductivity. This way, the applicator can be designed to adapt the cooling temperature to the desired treatment and the applied ingredients. For applications using higher temperatures, for example in the range from -10°C to +10°C, it is also possible to make the heat exchanger partly or wholly from a porous thermally conductive plastic.

The contact surface may have any suitable shape promoting easy application of the liquid. The contact surface may for example be convex.

The applicator may have any suitable shape. For example, it may have a dome shaped top aligned with a convex shape of the contact surface. The applicator may for example be cylindrical to allow easier insertion if the cutaneous surface to be treated is located in a body cavity, such as the user's nose, ear or anus.

In one embodiment, the applicator is provided with a child-proof lock to prevent unwanted actuation of the device.

In another embodiment the device is provided with a cover for the applicator covering the contact surface.

The applicator can be made removable from the container, the applicator having a substantially cylindrical skirt which is slidably guided on the outer surface of the container, but allowing the applicator to slide off from the container. If the applicator is not removable, the sleeve can be shorter, but there will be a stop preventing removal.

Refrigerant is to mean any liquid or gas which is able to extract heat due to evaporation and/or expansion or other cooling processes. The refrigerant can be selected depending on the particular application of the device and the required temperatures. An example of a suitable refrigerant is an aerosol containing butane/propane, but liquefied CO₂ or any other useful refrigerant can also be used. Also propellants that have a cooling effect, such as dimethyl ether, are considered to be a refrigerant.

The therapeutic or cosmetic ingredient can for example comprise a cosmetic additive, a pharmaceutical agent, a homoeopathic ingredient and/or an excipient. Optionally, the liquid may comprise further therapeutic and/or cosmetic ingredients.

The disclosed device is particularly suitable for the treatment of cutaneous disorders like insect bites, stings, burns, sunburn, wrinkles, eczema, skin irritations, nail or foot fungus, age spots, skin tags, swollen lips, bruises, swellings, nose bleeds or haemorrhoids. Such disorders can be treated non-surgically by epicutaneously cooling the disorder and simultaneous topical application of the therapeutic or cosmetic ingredient. This can be done by a professional practitioner, such as a physician or a nurse, or by the patient him/herself. In some cryogenic applications, for example with warts, the temperature of the device should be very low, in the order of-30°C to -50°C, in order to freeze the disorder. In other applications, for example with swellings, the temperature can be much higher, e.g., around 0°C.

The invention a further relates to a method of treating a cutaneous surface by applying a composition comprising a therapeutic or cosmetic ingredient and a refrigerant onto the cutaneous surface using a cooled contact surface, which is cooled by a porous heat exchanger through which the composition is passed during dispensing.

Further details and advantages of the invention will follow from the following description with reference to the accompanying drawings showing exemplary embodiments of the invention.
Figure 1 shows an exemplary embodiment of the device in cross section;
Figure 2 shows in side view the housing of the applicator of the device shown in Figure 1;
Figure 3 shows the housing of Figure 2 in bottom view;
Figure 4 shows the device of Figure 1 with a cover;
Figure 5 shows a bottom view of the applicator in a locked position;
Figure 6 shows a bottom view of the applicator in an unlocked position;
Figure 7 shows a top view of an alternative embodiment;
Figure 8 shows a longitudinal cross section of the device of Figure 7.

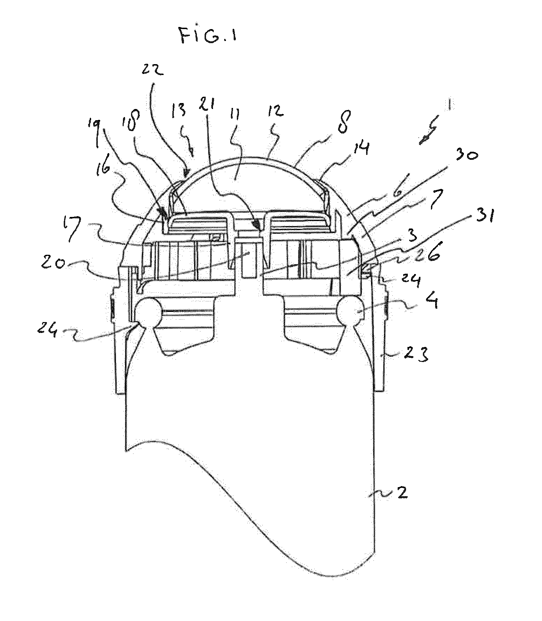

In the drawings Figure 1 shows a top section of an exemplary embodiment of a device 1 for the treatment of a cutaneous surface or mucous membrane, e.g., for medical or cosmetic purposes. The device 1 includes a container 2 containing a sprayable liquid composition comprising a refrigerant and a therapeutic or cosmetic ingredient. The container 2 may be a standard spray or aerosol can having a valve (not shown) and a container outlet tube 3. The can may have a capacity of 25 - 500 ml, more particular 50 - 100 ml, for example. The container 2 is mainly cylindrical having a collar 4 spaced coaxially around the container outlet 3.

An applicator 6 is mounted on the container 2 at the end with the container outlet 3. The applicator 6 comprises a dome shaped housing 7 and a convex contact surface 8 formed by a heat exchanger 9 held in the housing 7. The heat exchanger 9 comprises a porous body 11 of a thermally conductive material, such as a sintered metal alloy, and non-porous top layer 12 covering the porous body 11 at the contact surface 8. The non-porous layer 12 can for example be a rubber or plastic layer.

At its top end the housing 7 of the applicator 6 comprises an opening 13 exposing the contact surface 8. The opening 13 is bordered by a circumferential ridge 14 forming part of a fitting fixating the heat exchanger 11. The housing 7 of the applicator 6 comprises an inner cylindrical skirt 16 extending from the circumferential ridge 14 into the direction of the container 2.

The applicator 6 further comprises a bush 17 having one end connected to the container outlet 3 and one end engaging a lower surface the porous heat exchanger 11 opposite to the convex contact surface 8, so as to define a channel 20 between the container outlet 3 and the porous body 11 of the heat exchanger 9. At the outer end engaging the heat exchanger 9 the bush 17 comprises a collar 18 with a circumferential edge sealingly fitting into a recess 19 in the inner cylindrical skirt 16 of the applicator 6. The collar 18 is curved with its outer circumference curved downward, i.e., in the direction of the container 2, to form an inverse saucer-shape.

The bush 17 comprises an annular inner ridge 21 forming a support surface which rests on top of the container outlet 3. The valve in the container 2 is resiliently biased to push the container outlet tube 3 upward, i.e. towards the applicator 6. The container outlet tube 3 pushes the saucer-shaped collar 18 of the bush 17 against the heat exchanger 9, which in turn is pushed against the ridge 14 defining the opening 13 exposing the contact surface 8. This way, relative movement of the heat exchanger 9 and the applicator housing 6 is prevented.

The ridge 14 bordering the opening 13 exposing the contact surface 8 is profiled to define dispensing outlets 22 around the contact surface 8 for passage of dispensed liquid to wet the contact surface 8. The container outlet tube 3, the collared bush 17, the inner skirt 16 and the ridge 14 define a leaktight dispense channel between the container outlet tube 3 and the passages 22 around the contact surface 8. This channel and the non-porous top layer 12 fully enclose the porous body 11 to prevent contamination.

The applicator 6 comprises an outer skirt 23 extending slidably over a top edge of the container 2. In the shown embodiment, the outer skirt 23 is a separate cylindrical part connected to a lower edge of the applicator housing. In alternative embodiment the skirt may be an integral part of the applicator housing.

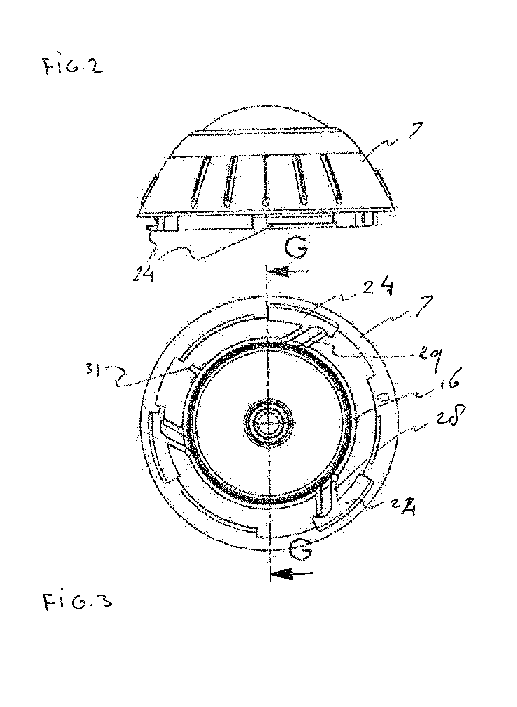

The applicator housing 7 is provided with three latches 24 extending in the direction of the container 2. The latches 24 form a snap connection with matching undercuts 26 near the upper inner edge of the outer skirt 23. As particularly shown in Figure 3, a bridge 28 extends between each of the latches 24 and the inner skirt 16 of the applicator housing 7.

Parts 29 are optional latches to lock the latches 24 and to prevent removal of the applicator housing 7 from the outer skirt 23.

The outer skirt 23 is provided with an inner latch 24 to form a snap connection with the collar 4 of the container 2. This snap connection is such that it allows up and down movement of the applicator 6 relative to the container 2 between an upper rest position and a lower dispensing position. This movement is guided by the slidable fit of the outer skirt 23 over the container 2.

The valve is resiliently biased into its closed position and pushes the container outlet tube 3, the collared bush 17, the heat exchanger 9 and the applicator housing 7 upwardly into the rest position.

The applicator 6 of the shown exemplary embodiment is provided with an optional child-proof lock to prevent the applicator from being activated, in this case depressed, by a child. The housing 7 may rotate to a limited extent with respect to skirt 23 around an axis which is in line with the axis of container 2.

In this embodiment, dome-shaped housing 7 is biased towards its closing position, so that a user must hold the dome-shaped wall portion 7 in its rotated position in order to be able to depress it. When the user releases the housing 7 it will spring back to its locked position.

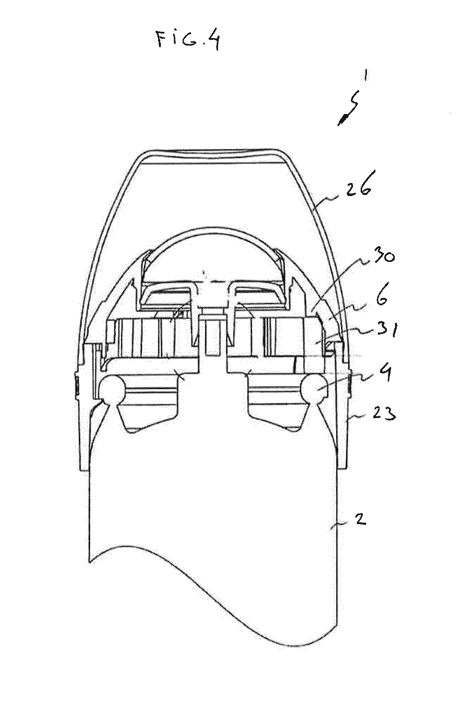

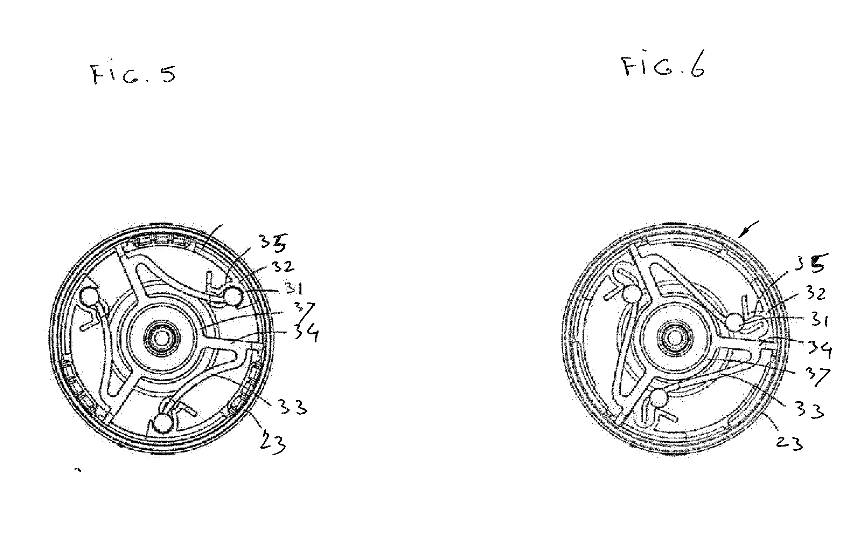

The child-proof lock in this embodiment comprises locking members in the form of locking cams 31, as shown in Figures 5 and 6 showing bottom views of the device 1 in a locked and unlocked position of the applicator 6, respectively. In the locking position of Figure 5, these cams 31 are positioned in seats 32 near the circumference of the housing 7. The cams 31 are then positioned above upper collar 4 of container 2 (see Figures 1 and 4), thereby preventing a downward movement of the applicator 6 because the cams 31 are stopped by the collar 4 of the container 2.

The housing 7 of the applicator 6 comprises downwardly protruding projections 30 (see Figures 1 and 4) engaging a respective cam 31 in axial direction. The cams 31 are mounted at free ends of transmitting or holding members, here flexible arms 33 which are curved and which are mounted at their ends remote from cams 31 to a part of the applicator 6 that is connected to skirt 23, in this case to radial ribs 34 extending between a central cylinder wall 37 and the skirt 23. The arms 33 extend mainly in circumferential direction and connect to cams 31 more in radial direction.

The seats 32 have a seat wall portion 35 mainly in radial direction on the side of arms 33. These seat wall portions 35 urge cams 31 radially inwardly when the seats 32 are moved mainly in circumferential direction upon rotation of the dome-shaped housing 7 and the arms 33 are held by the ribs 34 thereby pulling cams 31 away from their seats 32 to move along the seat wall portions 35 (see Figure 6). This inward, more or less radial movement of cams 31 causes cams 31 to move out of engagement with the collar 4, such that there is no part below cams 31 blocking their movement. The cams 31 and therewith dome-shaped housing 7 and skirt 23 may then be depressed with respect to container 2. As a result, outlet tube 3 of the container 2 may be depressed by a member of applicator 4, in this case bottom portion 13 of mounting member 12, and thus the valve of the container 2 is opened to allow liquid to enter the applicator 6. When the housing 7 is released, it will spring back upwardly by the spring pressure of outlet tube 3, while the spring pressure in arms 33 will rotate dome-shaped wall housing 7 back to the locked position with the cams 31 aligned with the collar 4 of the container 2.

In an alternative embodiment not shown, the spring force of the arms 33 may be reduced such that dome-shaped housing 7 will not be biased to the locked position. The dome-shaped housing 7 may then remain in the unlocked position and may be rotated back to the locked position manually. In such embodiment, wall portion 7 and skirt 23 may be provided with click members to mark the locked and unlocked position. It is conceivable to connect the holding and locking members to the housing 7 and to form the seats to the skirt 23, as long as there is a relative movement between the locking members and a part moving the locking member(s) away from the collar 4 of the container 2.

After unlocking the optional child-proof lock, a user can use the device 1 by moving the applicator 6 relative to the container 2 into the dispensing position. The collared bush 17 and the container outlet tube 3 are pushed in and actuate the valve to spray the liquid as an aerosol. The aerosol flows through the container outlet tube 3, the bush 17 and the porous body 11 and is dispensed via the outlet passages 22 around the contact surface 8. The contact surface 8 is wetted via the dispensing outlets 22. In the heat exchanger 9 the refrigerant component in the liquid extracts heat from the heat exchanger 9 which cools down. This cools the contact surface 8, enabling to provide a cold treatment combined with topical application of the therapeutic or cosmetic ingredient. The evaporated refrigerant escapes via the dispensing outlets 22 as a gas. The liquid remaining on the cooled contact surface 8 mainly comprises the therapeutic or cosmetic ingredients with other possible ingredients.

After a while, when a sufficient amount of liquid is collected on the cooled contact surface 8, the user may stop depressing applicator 6 so that it will return to its rest position by the resilient bias force of the valve of the container 2. The user can then apply the released liquid by massaging the cutaneous surface or mucous membrane with the cooled contact surface 8.

Figure 4 shows the applicator 6 capped by a cover 26 clicked onto the applicator to cover the contact surface 8. Also the cover may be provided with a child-proof lock, for example such that the cover and applicator can only be depressed after a small rotation of the cover and/or applicator. This can be effected by providing a member allowing depression of the applicator in one rotational position and preventing depression in another rotational position.

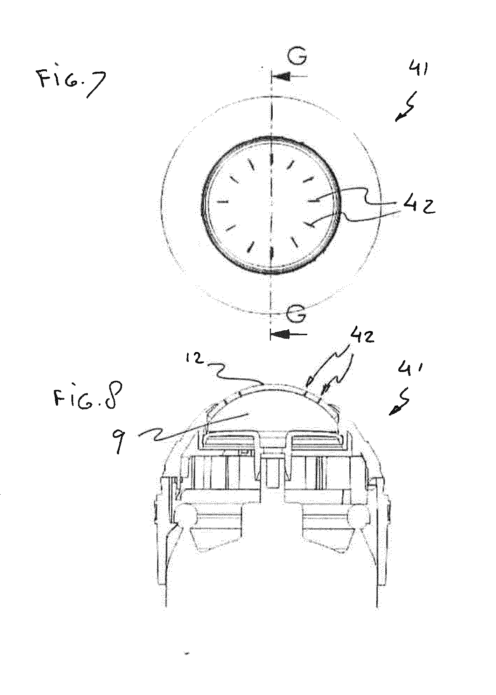

Figures 7 and 8 show an alternative embodiment of the device 41, which is identical to the device 1 of the preceding Figures, except that the non-porous layer 12 is provided with an array of radial slits 42. Same reference numbers are used for the same parts. When the applicator 6 is unlocked and depressed, the aerosol flows into the heat exchanger 9. Pressure in the porous body of the heat exchanger 9 increases and opens the slits 42, which form dispensing outlets for the released liquid and the evaporated refrigerant. After deactivation of the applicator 6, the slits 42 close again due to the elasticity of the non-porous layer 12 to isolate the porous body of the heat exchanger 9 during application the released liquid on the cutaneous surface or mucous membrane.

From the foregoing it will be clear that the invention provides a device which is very easy to handle and operate. The device can be easily controlled.

The invention is not limited to the embodiments shown in the drawings and described above which can be varied in different manners within the scope of the invention. First of all, it is noted that features of different embodiments can be used in other combinations. Furthermore, it is possible to replace parts of the device by alternative arrangements. For example, instead of a sintered porous heat exchanger, it is possible to use a member having one or more channels or passages formed therein allowing close contact between refrigerant and the heat exchanger material.

The mounting member and the wall portions of the applicator are generally made of plastic material. Especially the outer wall of the applicator is preferably made of material having a low heat conductivity (substantially lower than that of the porous heat exchanger), so that the temperature of the applicator housing does not lower too much.

The shape of the contact surface of the applicator may be varied in accordance with the disorder to be treated. In case of an applicator for treating piles in the anus, the applicator will have a narrower elongated part containing the contact member so that it can be inserted in the anus and the contact member surface will (also) be on the circumference of the elongated part, not (only) at the end thereof. In case the device is used for treatment in a body cavity, such as the nose or anus, disposable wrappers may be included with the device.

Generally, the applicator and the container will be a disposable unit. However, especially with the embodiment in which the applicator is removable from the container, it is possible to have a reusable applicator sold separately, so that if the container is empty it can be replaced by a new one to which the old applicator is mounted. Therefore the invention also covers an applicator without the container, which is adapted to be mounted to a fitting container. The valve for supplying refrigerant may also be present in the applicator.

## Claims

1. Device (1) for treatment of a cutaneous surface or a mucous membrane, the device comprising:
a container (2) for containing a composition comprising at least one refrigerant, the container having a container outlet (3) and a valve communicating with the container outlet,
an applicator (6) mounted or mountable to the container, the applicator comprising a contact surface (8) and a dispensing channel (20) between the container outlet and one or more applicator outlets (22) arranged for wetting the contact surface;
a thermo-conductive porous heat exchanger (9) defining the contact surface, the dispensing channel defining a flow path crossing the heat exchanger.

2. Device of claim 1, wherein the applicator (6) comprises a housing (7) with an opening (13) exposing the contact surface.

3. Device of claim 1 or 2, wherein the porous heat exchanger (9) comprises a non-porous layer (12) forming the contact surface.

4. Device of claim 3, wherein the non-porous layer (12) is made of a plastic or rubber material.

5. Device of any of the preceding claims, wherein the porous heat exchanger (9) is held by a ridge (14) of the applicator at least partly bordering the opening (13), the ridge being profiled to define the applicator outlets (22) at the circumference of the contact surface (8).

6. Device of claim 5, comprising a bush (16) having one end connected to the container outlet (3) and one end engaging the porous heat exchanger (9).

7. Device of claim 6, wherein the bush (17) comprises a support surface (21), e.g., an inner ridge (21) of the bush, resting on top of the container outlet (3).

8. Device of claim 7, the applicator being moveable between a rest position with the valve being closed, and a dispensing position with the support surface (21) of the bush pressing the container outlet (3) down to open the valve, wherein the valve resiliently biases the applicator (6) into the rest position.

9. Device of any of claims 6 - 8, wherein the end of the bush (17) engaging the heat exchanger comprises a collar (18) with a circumferential edge sealing against an inner cylindrical skirt (16) of the applicator.

10. Device of claim 9, wherein the collar (18) is curved to resiliently press against the porous heat exchanger (9).

11. Device of any of the preceding claims, wherein the applicator (6) comprises an outer skirt (23) extending over a top edge of the container (2).

12. Device of claim 11, comprising a snap joint (24) between the applicator (6) and the container (2) allowing up and down movement of the applicator relative to the container (2) between a rest position and a dispensing position.

13. Device of any preceding claim, wherein the porous heat exchanger (9) comprises a sintered material, such as metal like brass, or plastic.

14. Device of any preceding claim, wherein the composition comprises at least one therapeutic agent, at least one cosmetic ingredient and/or at least one excipient.

15. Method for treatment of a cutaneous surface or a mucous membrane by dispensing a composition comprising a therapeutic or cosmetic ingredient and a refrigerant onto the cutaneous surface or mucous membrane using an applicator comprising a contact surface (8), which is cooled by a porous heat exchanger (9) through which the composition is passed during dispensing.
